# EUROPEAN PATENT APPLICATION

(11) **EP 2 732 713 A1**
(43) Date of publication of application: **21.05.2014**
(21) Application number: 12877085.6
(22) Date of filing: 31.08.2012
(51) Int. Cl.: A24F 47/00

(54) **ELECTRONIC CIGARETTE WITH MULTIPLE TASTES**

(71) Applicant: Liu, Qiuming, Shenzhen, Guangdong 518000 (CN)
(72) Inventor: Liu, Qiuming, Shenzhen, Guangdong 518000 (CN)
(74) Representative: Beetz & Partner
(86) International application number: PCT/CN2012/080846
(87) International publication number: WO 2014/032276

(57) **Abstract**

This invention relates to a multi-flavored electronic cigarette, including liquid storage components adapted for reserving cigarette liquid and heaters adapted for atomizing cigarette liquid to smoke, the electronic cigarette being further provided with at least two atomizing chambers therein adapted for cigarette liquid being atomized therein into vapor mist, each atomizing chamber reserving a kind of cigarette liquid and provided with one heater, the electronic cigarette being further provided with a heater control circuit board therein electrically connected with each heater, a battery electrically connected with the heater control circuit board and a control switch disposed on an outer shell of the electronic cigarette. This kind of multi-flavored electronic cigarette has multiple atomizing chambers, and satisfies the requirement for free choice of one flavor, multi-flavors or a combination of optional flavors.

## Description

### TECHNICAL FIELD

This invention relates to a field of an electronic cigarette, and especially relates to a multi-flavored electronic cigarette.

### DESCRIPTION OF BACKGROUND

Current electronic cigarettes generally comprise a liquid storage component for storing cigarette liquid and a heater for atomizing cigarette liquid into vapor, and the electronic cigarettes also have one atomizing chamber for cigarette liquid to be atomized therein, while the atomizing chamber generally can only accommodate one flavor of cigarette liquid.

The current electronic cigarettes have the follow shortcomings: since only one atomizing chamber is provided for accommodating cigarette liquid, and each atomizing chamber generally can only accommodate one flavor of cigarette liquid, which results in that the vapor after atomized through the current electronic cigarettes has single taste, even cigarette liquid with multiple flavors are mixed in one atomizing chamber, all the flavors of the atomized smokes are also mixed together, this cannot satisfy user's requirement for free choice of multi-flavors and a combination of optional flavors.

### SUMMARY

An object of the present invention is to provide a multi-flavored electronic cigarette, which has multiple atomizing chambers and satisfies the requirement for free choice of one flavor, multi-flavors or a combination of optional flavors.

To solve the above object, the present invention provides a multi-flavored electronic cigarette, comprising liquid storage components adapted for reserving cigarette liquid and heaters adapted for atomizing cigarette liquid into vapor mist, the electronic cigarette being further provided with at least two atomizing chambers therein adapted for cigarette liquid being atomized therein into vapor mist, each atomizing chamber reserving a kind of cigarette liquid and provided with one heater, the electronic cigarette being further provided with a heater control circuit board therein electrically connected with each heater, a battery electrically connected with the heater control circuit board and a control switch disposed on an outer shell of the electronic cigarette.

Furthermore, the atomizing chambers each are provided with a conduit adapted for supporting the liquid storage components, the liquid storage components are sleeve-shaped, and fitted around outer portions of the conduits.

Furthermore, each of the heaters comprises a heating wire and a liquid guiding component adapted for absorbing cigarette liquid and supporting the heating wire, the conduit each defines locking slots through its side wall in a radial direction and adapted for mounting the liquid guiding component, and both ends of the liquid guiding component extend through the side wall of the conduit and abut against inner wall of the liquid storage components.

Furthermore, an inhaling tubethe inhaling tube is further provided with a cigarette liquid cup configured for receiving the liquid storage components and the heaters therein, the at least two atomizing chambers are defined in the cigarette liquid cup.

Furthermore, the cigarette liquid cup comprises a cup tube adapted for an inner wall of the inhaling tube, a cup holder with a vent hole therethrough, and a lid with vent hole therethrough; the lid and the cup holder are disposed at opposite ends of the cup tube; the cup tube defines at least two cavities; the lid is inserted into a top end of the main body and seals the cavities at the top; the cup holder is inserted into a bottom end of the cup tube and seal the cavities at the bottom; the lid and the cup holder are corresponding to restrain the opposite ends of the conduit; both the lid and the cup holder are corresponding and enclose sealed inner chambers served as the atomizing chamber together with side walls of the cavities; the liquid storage component is disposed within the atomizing chambers.

Furthermore, the cavities are cylinder-shaped; the lid comprises a circular main body, a positioning post axially extending from a bottom center of the main body to restrain the conduit, and a vent hole axially through both the position post and the main body; correspondingly, the cup holder comprises a circular main body, a positioning post axially extending from a central bottom center of the main body to restrain the conduit, and a vent hole axially through both the positioning post and the main body ; the positioning posts of the lid and the cup holder are respectively inserted into opposite ends of the conduit so as to mount the conduit in the atomizing chamber.

Furthermore, the electronic cigarette adopts a separated structure, and comprises an inhaling rod and a power rod interconnected, the inhaling rod at one end thereof is provided with a first connector, correspondingly, the power rod at one end thereof is provided with a second connector securely engaged with the first connector.

Furthermore, the heater and the cigarette liquid cup are disposed within the inhaling rod, while the battery and the heater control circuit board are disposed within the power rod; the first connector is provided with a first electrode of the heater and a first insulating sleeve therein, the first electrode of the heater is mounted by means of the first insulating sleeve into the first connector which serves as a second electrode of the heater; the second connector is provided with a first electrode of the battery and a second insulating sleeve therein; the first electrode of the battery is mounted by means of the second insulating sleeve into the second connector which serves as a second electrode of the battery.

Furthermore, the inhaling rod and the power rod are connected by fastener, plug or screw.

Furthermore, the electrode cigarette is configured as an integrated structure.

Furthermore, the liquid guiding component is made of glass fiber which is capable of absorbing water and reserving water like a sponge or a material with properties of absorbing water and isolating water.

Furthermore, the liquid storage components are made of high temperature resistant cotton, fiberglass cotton or thick cotton cloth capable of absorbing water and reserving water like a sponge.

The multi-flavored electronic cigarette of the present invention is provided with at least two atomizing chambers, each atomizing chamber reserves different flavored cigarette liquid; and it is available to depends on requirements to start the control switch for controlling the heater in the atomizing chamber to vapor cigarette liquid in the atomizing chambers; it is available to start the heater in a single atomizing chamber to work, or simultaneously start the heaters in all of the atomizing chambers to work, or start the heaters in any combination of optional atomizing chambers, to satisfy smoker with free choices of one flavor, multi-flavors or a combination of optional flavors.

The embodiments of the present invention will be described in further detail below in conjunction with the accompanying drawings.

### DESCRIPTION OF THE DRAWINGS

FIG. 1 is a cross-sectional view of a multi-flavored electronic cigarette in accordance with a first embodiment of the present invention.
FIG. 2 is an exploded view of the multi-flavored electronic cigarette in accordance with the first embodiment of the present invention.
FIG. 3 is a cross-sectional view of an inhaling rod of the multi-flavored electronic cigarette in accordance with the first embodiment of the present invention.
FIG. 4 is a cross-sectional view of a cup holder of the inhaling rod of the multi-flavored electronic cigarette in accordance with the first embodiment of the present invention.
FIG. 5 is a cross-sectional view of a lid of the inhaling rod of the multi-flavored electronic cigarette in accordance with the first embodiment of the present invention.
FIG. 6 is a cross-sectional view of a cup tube of the inhaling rod of the multi-flavored electronic cigarette in accordance with the first embodiment of the present invention.

### DETAILED DESCRIPTION OF THE EMBODIMENTS

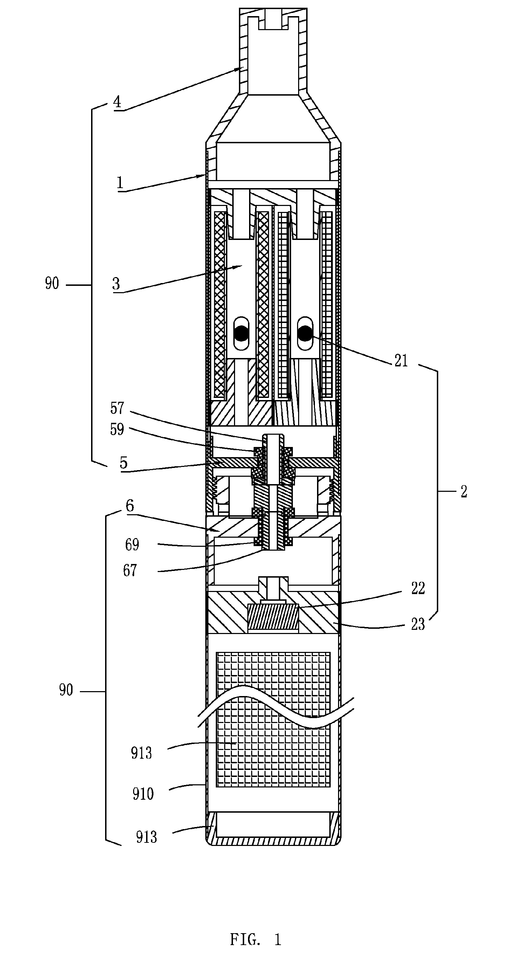

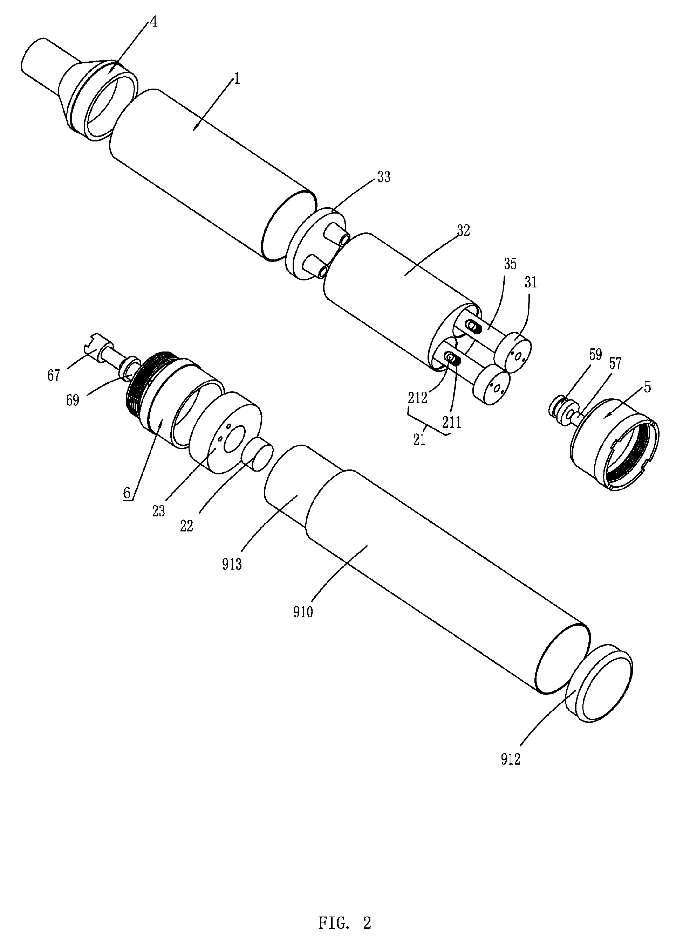

As shown in FIGS. 1-6, a first embodiment of the present invention provides a multi-flavored electronic cigarette 100, the multi-flavored electronic cigarette 100 is a separated electronic cigarette, which comprises an electronic cigarette inhaling rod 90 and a power rod 91, the electronic cigarette inhaling rod 90 and the power rod 91 are connected by fastener, plug or screw, and in this embodiment by screw, and the electronic cigarette inhaling rod 90 and the power rod 91 are screwed together by a first connector 5 disposed in the inhaling rod 90 and a second connector 6 disposed in the power rod 91 described in details hereafter. The directions described in this embodiment are based on the directions shown in FIG. 2.

In accordance with the embodiment, the electronic cigarette inhaling rod 90 comprises an inhaling tube 1 with a hollow inner chamber, a heating device 2, a cigarette liquid cup 3 defining multiple atomizing chambers therein, a mouthpiece 4 with a vent hole therethrough, and the first connector 5 for connecting with the power rod 9. The mouthpiece 4 and the first connector 5 are respectively mounted at opposite ends of the inhaling tube 1, and the heating device 2 and the cigarette liquid cup 3 are disposed within the inhaling tube.

As shown in FIG. 2 to FIG. 6, the heating device 2 comprises a heater 21, a heater control circuit board 22 and a circuit board positioning seat 23 for positioning the heater control circuit board 22; in accordance with this embodiment, the heater 21 is disposed within the inhaling rod 90 of the electronic cigarette, while the heater control circuit board 22 and the circuit board positioning seat 23 are disposed within the power rod 91; the heater control circuit board 22 respectively controls multiple heaters 21 to work, and can simultaneously start them or any combination of several of them. In accordance with the embodiment, two heaters 21 are provided.

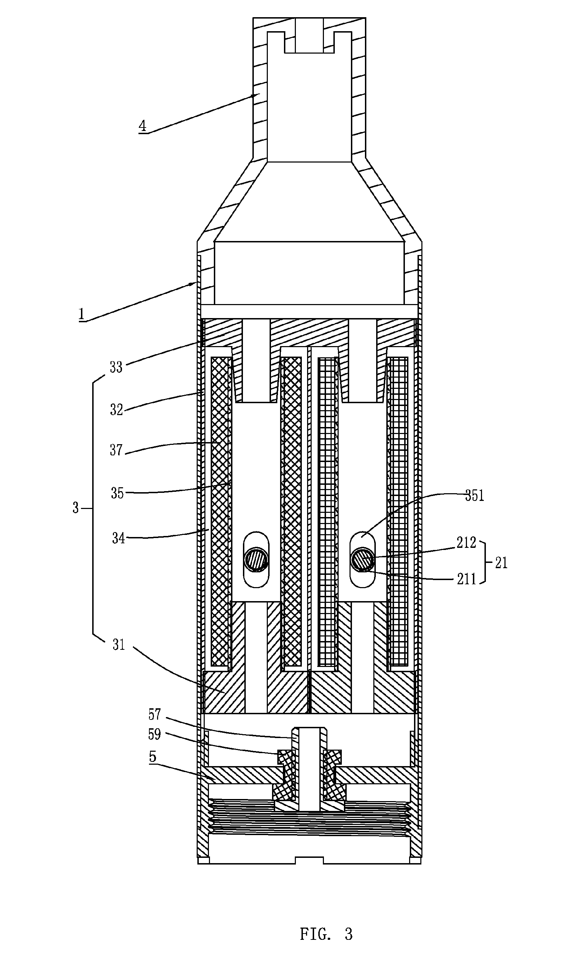

As shown in FIG. 3, the heater 21 provided for atomizing cigarette liquid into vapor mist, comprises a heating wire 211 and a liquid guiding component 212 provided for absorbing cigarette liquid and supporting the heating wire 211; the heating wire 211 winds around the liquid guiding component 212; and the liquid guiding component 212 can absorb and reserve water like a sponge, and is made of glass fibers or a material with properties of absorbing water and isolating water, such as a cotton material. In accordance with this embodiment, the liquid guiding component 212 is fixed within the cigarette liquid cup 3; opposite ends of the heating wire 211 extend out of the cigarette liquid cup 3 and then electrically connect with a positive electrode and a negative electrode of the power rod 91.

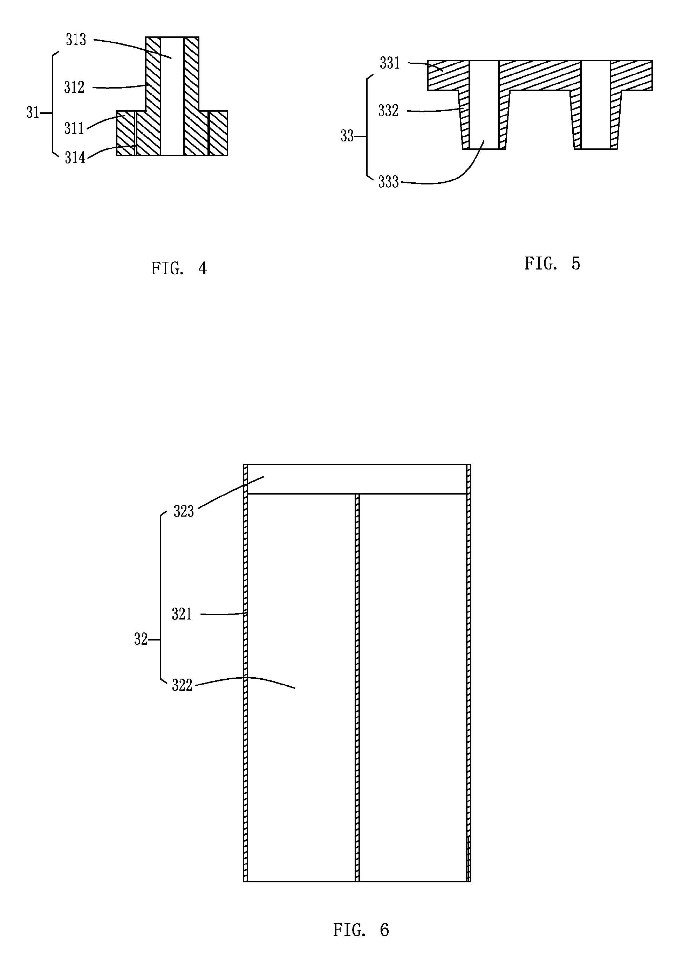

As shown in FIG. 3 and FIG. 4, in accordance with the embodiment, the cigarette liquid cup 3 comprises cup holders 31, a cup tube 32, a lid 33, conduits 35 and liquid storage components 37; the cigarette liquid cup 3 defines the atomizing chambers 34 therein for cigarette liquid being vaporized therein. Wherein, the cup holders 31 and the lid 33 are disposed at opposite ends of the cup tube 32, the conduits 35 are secured between the cup holder 31 and the lid 33; the liquid storage components 37 are fitted around the conduits 35 and disposed between the cup holder 31 and the lid 33.

The cup holder 31 (referring to FIG. 3 and FIG. 4) comprises a circular main body 311, a positioning post 312 axially extending from a central portion of a top surface of the main body 311, and a vent hole 313 axially through both the positioning post 312 and the main body 311; the cup holder 31 further defines perforations 314 for passing wires of the heating wires 211 therethrough. The main body 311 is tightly fitted in an inner wall of a cavity 322 in the cup tube 32 which will be described in details hereafter. The cup holder 31 may be made from silicone material.

The cup tube 32 (referring to FIG. 3 and FIG. 6) is cylindrical, and comprises a cylindrical main body 321 defining the cavities 322 axially through the main body 321 and a through hole 323 in top of the main body 321; the cup tube 32 defines at least two cavities 322 therein used for receiving the liquid storage components 37 and the heaters 21; there are two cavities 322 in accordance with this embodiment, both the two cavities 322 are communicated with the through hole 323, and each cavity 322 reserves different flavor of cigarette liquid. The main body 321 is tightly fitted in an inner wall of the inhaling tube 1 by means of its sidewall. The through hole 323 is used for mounting the lid 33.

The lid 33 (referring to FIG. 3 and FIG. 5) may be made from silicone material, in accordance with the embodiment, it comprises a circular main body 331, positioning posts 332 axially extending from a central portion the main body 331, and vent holes 333 axially through both the position posts 332 and the main body 331; the lid 33 has its outer diameter a little bigger that an inner diameter of the through hole 323 in the cup tube 32, and the lid 33 is secured in an inner wall of the through hole 323 by means of its sidewall. When cigarette liquid in the cigarette liquid cup 3 is used out, necessary cigarette liquid can be refilled into the atomizing chambers 34 of the cigarette liquid cup 3 after removing the lid 33. The lid 33 in accordance with the embodiment is adaptive for the main body 1, and forms two positioning posts 332 thereon used for engaging with the two cavities 322, the positioning posts 41 of the lid 33 correspond to the positioning posts 311 of the cup holder 31, and respectively fix opposite ends of the conduits 35. The bottom wall of the main body 331 of the lid 33 and the top wall of the main bodies 311 of the cup holder 31 are corresponding, and together with the sidewall of the cavities 322, enclose inner chambers to be used as the atomizing chambers 34; the liquid storage components 37 are received in the atomizing chambers 34.

The conduit 35 (referring to FIG. 2 and FIG. 3) is used for supporting the liquid storage component 37 and supporting the liquid guiding component 212, and further serve as a channel for passing vapor mist to outside of the inhaling tube 1 after the heater 21 atomizes cigarette liquid into vapor mist. The conduit 35 in accordance with this embodiment is a hollow circular pipe, and may be made from plastic or fiber materials, such as glass fiber, comprising a top portion and a bottom portion; the top portion is sleeved around the positioning posts 41 of the lid 33 with a peripherally sealed connection, while the bottom portion is sleeved around the positioning posts 331 of the cup holders 31 with a peripherally sealed connection. The conduit 35 defines locking slots 351 through its sidewall for supporting and fixing the liquid guiding component 212; the liquid guiding component 212 crosses the conduit 35 with both ends thereof respectively passing through the locking slots 351 and abutting against the liquid storage component 37 so as to absorb cigarette liquid for atomization by the heating wires 211.

The liquid storage component 37 (referring to FIG. 2 and FIG. 3) is used for absorbing and storing cigarette liquid in the cigarette liquid cup 3 for the heater 2 to atomize cigarette liquid, can absorb and reserve water like a sponge, and may be made from a material with properties of absorbing water and isolating water, such as a cotton material. The liquid storage component 37 has a hollow tube structure, is sleeved around an outer side of the conduits 35 and tightly fitted on the outer wall of the conduits 35 so as to be supported, and received in the cavity 34. The liquid storage component 37 at its sidewall is contacted by the liquid guiding component 212, thereby cigarette liquid is delivered into and absorbed by the liquid guiding component 212 from the liquid storage component 37, and then is vaporized into mist by the heating wires 211.

As shown in FIG. 1 and FIG. 3, the first connector 5 has a shape adaptive for the inhaling tube 1, and is made from conductive material, such as iron material. The first connector 5 is substantially a hollow cylinder, and comprises a cylindrical main body and a positioning flange radically and outwardly extending from the main body; the main body is inserted into the inhaling tube 1 to be positioned by means of the positioning flange being securely engaged with the inhaling tube 1; the first connector 5 on its inner wall forms inner threads engaging with the second connector 6; and is further provided with a locking ring at its inner wall. In accordance with the embodiment, the first connector 5 serves as a second electrode (such as positive electrode) of the heater 21. The first connector 5 is provided with a first electrode 57 (such as negative electrode) of the heater and a first insulating sleeve 59 therein; the first electrode 57 of the heater defines a vent hole through its center, the first electrode 57 of the heater is fixed in the locking ring and insulated from the first connector 5 by means of the first insulating sleeve 59.

As shown in FIG. 2 and FIG. 3, the power rod 91 comprises a sheath 910, the second connector 6 and an end cap 912 respectively disposed at opposite ends of the sheath 910, and a battery 913 received in the sheath 910.

As shown in FIG. 1 and FIG. 2, the second connector 6 and the first connector 5 are matched; the second connector 6 is disposed at a top end of the sheath 910, is used for connecting the inhaling tube 90 with the power rod 91, and is made from conductive materials, such as iron material; the second connector 6 is substantially a hollow cylinder, and comprises a cylindrical upper portion and a cylindrical lower portion, the upper portion externally forms outer threads for threadedly engaging with the inner threads of the first connector 5; a positioning step is provided between the upper portion and the lower portion for matching with the sheath 910, the second connector 6 is secured to the inner wall of the sheath 910 by expansion of its bottom outer wall. The second connector 6 is provided with a locking ring at its inner wall. The second connector further is provided with a first electrode 67 of the battery therein used for being electrically connected to an electrode (such as negative electrode) of the battery 913 and a second insulating sleeve 69, the first electrode 67 of the battery forms a vent hole in a central portion thereof, and the first electrode 67 of the battery is mounted to the locking ring of the second connector by means of the second insulating sleeve 69 and insulated from the second connector 6. In accordance with the embodiment, the second connector 6 serves as a second electrode (such as positive electrode) of the battery 913.

Each atomizing chamber 34 is provided with one liquid storage component 37 therein for reserving cigarette liquid of one kind flavor, and each atomizing chamber 34 is correspondingly provided with one heater 21 therein; each heater 21 is connected to the heater control circuit board 22, and is controlled via a control switch (not shown); the control switch is disposed on an outer wall of the electronic cigarette, for starting the heater 21 in the atomizing chamber 34 corresponding to the required flavor of cigarette liquid. Before the electronic cigarette works, a small amount of cigarette liquid from the liquid storage component 37 has penetrated and been stored in the liquid guiding component 212; at work, the control switch is pressed according to the required flavor of cigarette liquid, the heater control circuit board 22 controls the heater 21 in the corresponding atomizing chamber 34 to work, the heating wire 211 is electrified to generate heat, cigarette liquid in the liquid guiding component 212 is thus atomized into vapor mist; the vapor mist passes through the conduit 5 and the lid 33 to an exterior of the cigarette liquid cup 3 and flows into the inner chamber of the inhaling tube 1, and finally passes through the vent hole in the mouthpiece 4 to be inhaled by smoker. Since each atomizing chamber is provided with one heater 21 to atomize cigarette liquid reserved therein, cigarette liquid in each atomizing chamber 34 is atomized into vapor mist to get into the inner chamber of the inhaling tube 1; smoker can start the heater 21 in single atomizing chamber 34 via the control switch to atomize the required flavor of cigarette liquid according to his desire, by which option of single, multiple or combination of flavor(s) of cigarette liquid is available. When multiple flavors are desired, cigarette liquid of each flavor is respectively atomized by the heater 21 in its own atomizing chamber 34, and then aerosol of cigarette liquid with various flavors is mixed in the inhaling tube 1 and is inhaled by the smoker.

The electronic cigarette of the present invention, defines two atomizing chambers 34, capable of providing two different flavors of cigarette liquid for smokers to choose; certainly, also can define three or more atomizing chambers 34 according to client need, the amounts of other related components cooperated with the atomizing chambers 34 are adaptively adjusted. The electronic cigarette 100 in accordance with the embodiment of the present invention adopts a separated structure; understandably, the electronic cigarette 100 can also adopt an integrated structure, by which the inhaling tube and the power rod are integrally formed.

The above-mentioned is only the embodiments of the present invention. It should be noted, for the persons of ordinary skill in this field, improvements and modifications within the spirit of the present invention can be made, and the improvements and modifications should be seemed to be included in the claimed scope of this invention.

## Claims

1. A multi-flavored electronic cigarette, comprising liquid storage components adapted for reserving cigarette liquid and heaters adapted for atomizing cigarette liquid to smoke, the electronic cigarette being further provided with at least two atomizing chambers therein adapted for cigarette liquid being atomized therein into vapor mist, each atomizing chamber reserving a kind of cigarette liquid and provided with one heater, the electronic cigarette being further provided with a heater control circuit board therein electrically connected with each heater, a battery electrically connected with the heater control circuit board and a control switch disposed on an outer shell of the electronic cigarette.

2. The multi-flavored electronic cigarette as described in claim 1, wherein, the atomizing chambers each are provided with a conduit adapted for supporting the liquid storage components, the liquid storage components are sleeve-shaped, and sleeved around outer portions of the conduits.

3. The multi-flavored electronic cigarette as described in claim 2, wherein, each of the heaters comprises a heating wire and a liquid guiding component adapted for absorbing cigarette liquid and supporting the heating wire, the conduits each form a locking slot extending through a side wall thereof in a radial direction and adapted for mounting the liquid guiding component, and both ends of the liquid guiding component extend through the inner wall of the conduit and abut against inner wall of the liquid storage components.

4. The multi-flavored electronic cigarette as described in any one of claims 1~3, wherein, an inhaling tubethe inhaling tube is further provided with a cigarette liquid cup configured for receiving the liquid storage components and the heaters therein, the at least two atomizing chambers are defined in the cigarette liquid cup.

5. The multi-flavored electronic cigarette as described in claim 4, wherein, the cigarette liquid cup comprises a cup tube adapted for an inner wall of the inhaling tube, a cup holder with an vent hole therethrough, and a lid with a vent hole therethrough; the lid and the cup holder are disposed at opposite ends of the cup tube; the cup tube defines at least two cavities therein; the lid is inserted into a top end of the main body and seals the cavities at the top, the cup holder is inserted into a bottom end of the cup tube and seals the cavities at the bottom; the lid and the cup holder are corresponding to restrain opposite ends of the conduit; the lid and the cup holder are corresponding and construct sealed inner chambers served as the atomizing chambers together with side walls of the cavities, the liquid storage components are disposed within the atomizing chambers.

6. The multi-flavored electronic cigarette as described in claim 5, wherein, the cavities are cylinder-shaped, the lid comprises a circular main body, a positioning post axially extending from a bottom center of the main body to restrain the conduit, and a vent hole axially through both the position post and the main body; correspondingly, the cup holder comprises a circular main body, a positioning post axially extending from a bottom center of the main body to restrain the conduit, and a vent hole axially through both the positioning post and the main body ; the positioning posts of the lid and the cup holder are respectively inserted into opposite ends of the conduit to mount the conduit in the atomizing chamber.

7. The multi-flavored electronic cigarette as described in claim 5, wherein, the electronic cigarette adopts a separated structure, and comprises an inhaling rod and a power rod interconnected; the inhaling rod at one end thereof is provided with a first connector, correspondingly, the power rod at one end thereof is provided with a second connector securely engaged with the first connector.

8. The multi-flavored electronic cigarette as described in claim 7, wherein, the heater and the cigarette liquid cup are disposed within the inhaling rod, while the battery and the heater control circuit board are disposed within the power rod; the first connector is provided with a first electrode of the heater and a first insulating sleeve therein, the first electrode of the heater is mounted by means of the first insulating sleeve into the first connector which serves as a second electrode of the heater; the second connector is provided with a first electrode of the battery and a second insulating sleeve therein; the first electrode of the battery is mounted by means of the second insulating sleeve into the second connector which serves as a second electrode of the battery.

9. The multi-flavored electronic cigarette as described in claim 5, wherein, the inhaling rod and the power rod are connected by fastener, plug or screw.

10. The multi-flavored electronic cigarette as described in claim 5, wherein, the electrode cigarette is configured as an integrated structure.

11. The multi-flavored electronic cigarette as described in claim 3, wherein, the liquid guiding component is made of glass fiber which is capable of absorbing water and reserving water like a sponge or is made of a material with properties of absorbing liquid and isolating liquid.

12. The multi-flavored electronic cigarette as described in claim 1, wherein, the liquid storage components are made of high temperature resistant cotton, fiberglass cotton or thick cotton cloth capable of absorbing water and reserving water like a sponge.
